# EUROPÄISCHE PATENTSCHRIFT

(11) **EP 2 328 501 B1**
(45) Veröffentlichungstag und Bekanntmachung des Hinweises auf die Patenterteilung: **25.03.2020**
(21) Anmeldenummer: 09777975.5
(22) Anmeldetag: 19.08.2009
(51) Int. Cl.: A61B 34/30, A61B 34/37, A61B 34/00

(54) **MEDIZINISCHER ARBEITSPLATZ UND BEDIENVORRICHTUNG ZUM MANUELLEN BEWEGEN EINES ROBOTERARMS EINES MEDIZINISCHEN ARBEITSPLATZES**
MEDICAL WORK STATION AND OPERATING DEVICE FOR MANUALLY MOVING A ROBOT ARM OF A MEDICAL WORK STATION
POSTE DE TRAVAIL MÉDICAL ET DISPOSITIF DE COMMANDE POUR DÉPLACER MANUELLEMENT UN BRAS ROBOTISÉ D UN POSTE DE TRAVAIL MÉDICAL

(30) Priorität: 29.08.2008 DE 102008041709
(43) Veröffentlichungstag der Anmeldung: 08.06.2011
(73) Patentinhaber: KUKA Deutschland GmbH, 86165 Augsburg (DE)
(72) Erfinder: ORTMAIER, Tobias, 30966 Hemmingen (DE)
(74) Vertreter: Böss, Dieter Alexander
(86) Internationale Anmeldenummer: PCT/EP2009/006010
(87) Internationale Veröffentlichungsnummer: WO 2010/022884

(56) Entgegenhaltungen:
- WO-A-98/13783
- US-A1- 2003 109 780
- US-A1- 2006 087 746
- US-A1- 2006 142 657
- US-A1- 2008 046 122
- US-B1- 6 911 916

## Beschreibung

Die Erfindung betrifft einen medizinischen Arbeitsplatz und eine Bedienvorrichtung zum manuellen Bewegen eines Roboterarms eines medizinischen Arbeitsplatzes.

Die EP 0 883 376 B1 offenbart einen medizinischen Arbeitsplatz mit mehreren zum Behandeln eines Patienten vorgesehenen Roboterarmen, die mittels einer Bedienvorrichtung des medizinischen Arbeitsplatzes manuell bewegt werden können. Die Bedienvorrichtung umfasst eine Steuereinrichtung und mit dieser gekoppelte manuell bewegbare erste und zweite Eingabeeinrichtungen. Werden die Eingabeeinrichtungen bewegt, so erzeugt die Steuereinrichtung ein Signal, um die Roboterarme entsprechend zu bewegen.

Die US 2006/0087746 A1 offenbart eine Bedienvorrichtung mit einer Eingabevorrichtung zum manuellen Bewegen eines Roboterarms, mit dem ein Patient behandelt werden kann. Die Bedienvorrichtung ist eingerichtet, dass an diese dem Patienten zugeordnete Daten übermittelt werden können. Diese Daten werden an ein Netzwerk übermittelt und die Bedienvorrichtung umfasst eine Schnittstelle, über die die Bedienvorrichtung an das Netzwerk angeschlossen werden kann.

Die US 6,911,916 B1 offenbart einen medizinischen Arbeitsplatz mit einem Roboterarm, der über einen Slave Controller mit einem Master Controller verbunden ist. Der Master Controller stellt eine Benutzerschnittstelle dar. Um Geräte, wie z.B. den Roboterarm, leichter bedienen zu können, weist die Benutzerschnittstelle eine Spracheingabe auf. Einer der Slave Controller kann mit einem Krankenhausdatennetz verbunden werden kann, sodass mittels des Master Controllers von dem zu behandelnden Patienten zugeordnete Daten zur Verfügung stehen.

Die US 2006/0142657 A1 offenbart einen medizinischen Arbeitsplatz mit einem Roboterarm, welcher mit einem Computersystem verbunden ist. Der Computer des Computersystems ist eingerichtet, Befehle an den Steuerrechner für den Roboterarm zu übermitteln, welche mittels Eingabemittel eingegeben werden können.

Die Aufgabe der Erfindung ist es, Voraussetzungen für eine flexiblere Bedienvorrichtung für einen solchen medizinischen Arbeitsplatzes anzugeben.

Die Aufgabe der Erfindung wird gelöst durch eine Bedienvorrichtung zum manuellen Bewegen eines Roboterarms eines medizinischen Arbeitsplatzes, aufweisend eine Steuerungsvorrichtung, die eingerichtet ist, ein zum Steuern einer Bewegung eines zum Behandeln eines Lebewesens vorgesehenen ersten Roboterarms vorgesehenes erstes Signal zu erzeugen, eine mit der Steuerungsvorrichtung gekoppelte manuelle erste Eingabevorrichtung, wobei die Steuerungsvorrichtung das erste Signal aufgrund eines manuellen Bewegens der ersten Eingabevorrichtung erzeugt, so dass der erste Roboterarm eine des manuellen Bewegens entsprechende Bewegung durchführt, und einen Bildschirm, wobei die Steuerungsvorrichtung eine Schnittstelle aufweist, die vorgesehen ist, mit einem Krankenhausdatennetz verbunden zu werden und die Steuerungsvorrichtung eingerichtet ist, dem Lebewesen zugeordnete, vor einer Behandlung des Lebewesens mit dem ersten Roboterarm erstellte Daten über die Schnittstelle und dem Krankenhausdatennetz abzurufen und den Daten zugeordnete Informationsinhalte am Bildschirm darzustellen, und die Steuerungsvorrichtung und deren erste Eingabevorrichtung eingerichtet sind, die am Bildschirm dargestellten Informationsinhalte mittels der ersten Eingabevorrichtung zu ändern.

Ein weiterer Aspekt der Erfindung betrifft einen medizinischen Arbeitsplatz, der die erfindungsgemäße Bedienvorrichtung und wenigstes einen zum Behandeln des Lebewesens vorgesehenen medizinischen Roboterarm aufweist, dessen Bewegung mittels der Bedienvorrichtung manuell steuerbar ist.

Die erfindungsgemäße Bedienvorrichtung bzw. der erfindungsgemäße medizinische Arbeitsplatz sind dafür vorgesehen, dass z.B. ein Arzt das Lebewesen mittels eines oder mehrere Roboterarme behandelt. Der Roboterarm kann bzw. die Roboterarme können beispielsweise mit einem medizinischen Instrument, insbesondere mit einem minimalinvasiven medizinischen Instrument versehen sein, wobei der Arzt den Roboterarm bzw. die Roboterarme und somit das medizinische Instrument mittels der manuellen Eingabevorrichtung bewegt. Der Roboterarm kann beispielsweise in sechs Freiheitsgraden bewegt werden. Die Eingabevorrichtung kann z.B. dieselbe Anzahl von Freiheitsgraden wie oder mehr Freiheitsgrade als der zu bewegenden Roboterarm aufweisen. Aufgrund des manuellen Bewegens des Roboterarms mittels der Eingabevorrichtung ergibt sich ein relativ einfaches manuelles Bewegen des Roboterarms bzw. der Instrumentenspitze. Das medizinische Instrument, insbesondere wenn in der minimal invasiven Chirurgie eingesetzt, kann ebenfalls aktuiert sein, d.h. über eigene, mit Motoren, Getriebe, etc. versehene Antriebe umfassen.

Die erfindungsgemäße Bedienvorrichtung weist neben der ersten Eingabevorrichtung die mit dieser gekoppelten Steuerungsvorrichtung auf. Aufgrund des Bewegens der ersten Eingabevorrichtung erzeugt die Steuerungsvorrichtung das erste Signal, mittels dessen die Bewegung des ersten Roboterarms entsprechend des Bewegens der ersten Eingabevorrichtung gesteuert wird. Die Steuerungsvorrichtung kann z.B. direkt den ersten Roboterarm ansteuern oder mit einer weiteren Steuerungsvorrichtung verbunden sein, die Antriebe zum Bewegen des ersten Roboterarms ansteuert. Es kann auch eine Verarbeitung des ersten Signals, zum Beispiel eine Filterung und/ oder Skalierung, vorgenommen werden.

Die erfindungsgemäße Bedienvorrichtung kann aber auch eine manuelle zweite Eingabevorrichtung aufweisen, mittels derer die Bewegung eines zweiten Roboterarms des medizinischen Arbeitsplatzes manuell gesteuert werden kann. Somit wird es dem das Lebewesen behandelnden Arzt erlaubt, die Bewegung zweier Roboterarme gleichzeitig manuell zu steuern. Die zweite Eingabevorrichtung kann insbesondere genauso viele Freiheitsgrade wie oder mehr Freiheitsgrade als der zweite Roboterarm aufweisen.

Die erfindungsgemäße Bedienvorrichtung umfasst ferner den Bildschirm, der insbesondere vorgesehen ist, eine Bewegung des ersten Roboterarm und/oder gegebenenfalls des zweiten Roboterarms und/ oder der den Roboterarmen zugeordneten Instrumente darzustellen. Ist z.B. am Roboterarm eine Kamera befestigt oder umfasst das am Roboterarm befestigte medizinische Instrument eine Kamera, dann ist der Bildschirm vorgesehen, mit dieser Kamera aufgenommene Bilder darzustellen.

Die Steuerungsvorrichtung der erfindungsgemäßen Bedienvorrichtung weist ferner die Schnittstelle auf, über die die erfindungsgemäße Bedienvorrichtung bzw. deren Steuerungsvorrichtung mit dem Krankenhausdatennetz verbunden werden kann. Das Krankenhausdatennetz ist z.B. mit einem so genannten PACS verbunden, so dass der das Lebewesen behandelnde Arzt dem Lebewesen zugeordnete und z.B. im DICOM-Format vorliegende Daten über die Schnittstelle mittels der erfindungsgemäßen Bedienvorrichtung abrufen kann, so dass die den Daten zugeordneten Informationsinhalte mit dem Bildschirm der erfindungsgemäßen Bedienvorrichtung dargestellt werden. Die Daten, die vor einer Behandlung des Lebewesens mit dem ersten Roboterarm erstellt wurden, sind z.B. der Krankenakte des Lebewesens zugeordnet und/oder die Informationsinhalte umfassen beispielsweise Bilder, insbesondere mit einem bildgebenden medizinischen Gerät aufgenommene Bilder des Lebewesens, wie z.B. Röntgen-, Ultraschall- oder Magnetresonazbilder. Somit wird es dem Arzt in relativ einfacher Weise ermöglicht, während der Behandlung des Lebewesens mit dem Roboterarm dem Lebewesen zugeordnete Informationen in relativ einfacher abzurufen und am Bildschirm darzustellen.

Die Steuerungsvorrichtung und die erste Eingabevorrichtung eingerichtet, die am Bildschirm dargestellten Informationsinhalte mittels der ersten Eingabevorrichtung zu ändern und/oder mittels eines Betätigens der ersten Eingabevorrichtung die Daten über die Schnittstelle abzurufen. Die erfindungsgemäße Bedienvorrichtung umfasst die erste Eingabevorrichtung, die ähnlich Eingabevorrichtungen konventioneller Bedienvorrichtungen zum Bewegen des ersten Roboterarms vorgesehen ist. Da gemäß dieser Variante der erfindungsgemäßen Bedienvorrichtung zusätzlich die erste Eingabevorrichtung ausgeführt ist, die am Bildschirm dargestellten Informationsinhalte zu ändern bzw. die Daten über die Schnittstelle abzurufen, werden keine weiteren Eingabemittel für eine solche Änderung bzw. ein solches Abrufen, wie z.B. eine Rechnermaus, benötigt. Insbesondere ist kein weiterer Arbeitsplatz zum Abrufen und Betrachten der dem Patienten zugeordneten Daten notwendig. Dies stellt für den Operateur eine erhebliche Erleichterung des Operierens dar, da er den Arbeitsplatz zum Betrachten der Daten nicht verlassen und die Operation nicht unterbrechen muss. Des Weiteren geht eine Reduktion der Kosten einher. Unter einer Änderung der dargestellten Informationsinhalte wird u.A. eine Änderung deren Darstellung, z.B. ein Navigieren innerhalb der Informationsinhalte, und/oder eine Änderung der den Informationsinhalten zugeordneten Daten verstanden. Insbesondere handelt es sich auch um eine Überlagerung der Patientendaten mit den Daten der Kamera um spezielle anatomische Strukturen geeignet darzustellen. Diese Technik ist unter dem Begriff "Augmented Reality" dem Fachmann bekannt. Spezielle Strukturen können zum Beispiel im Kamerabild verdeckte, nur auf den dem Patienten zugeordneten Daten sichtbare Blutgefäße sein, deren versehentliche Verletzung zu vermeiden ist.

Die erfindungsgemäße Bedienvorrichtung bzw. der erfindungsgemäße medizinische Arbeitsplatz kann eine mit der Steuerungsvorrichtung gekoppelte manuelle zweite Eingabevorrichtung aufweisen, wobei die Steuerungsvorrichtung eingerichtet ist, aufgrund eines manuellen Bewegens der zweiten Eingabevorrichtung ein zweites Signal zu erzeugen, aufgrund dessen ein zur Behandlung des Lebewesens vorgesehener medizinischer zweiter Roboterarm eine des manuellen Bewegens der zweiten Eingabevorrichtung entsprechenden Bewegung durchführt, wobei die Steuerungsvorrichtung und die zweite Eingabevorrichtung eingerichtet sind, die am Bildschirm dargestellten Informationsinhalte mittels der zweiten Eingabevorrichtung zu ändern. Aufgrund dieser Variante ist es möglich, dass der das Lebewesen behandelnde Arzt mit beiden Eingabeeinrichtungen die dargestellten Informationsinhalte ändern kann. Die Steuerungsvorrichtung kann z.B. direkt die Roboterarme ansteuern oder mit einer weiteren Steuerungsvorrichtung verbunden sein, die Antriebe zum Bewegen der Roboterarme ansteuert. Im zweiten Fall kann, wenn mehrere Roboterarme verwendet werden, eine einzige Steuerungsvorrichtung alle oder mehrere der Roboterarme gemeinsam direkt ansteuern oder es kann jedem einzelnen Roboterarm eine individuelle Steuerungsvorrichtung zugeordnet sein, die von der Steuervorrichtung der erfindungsgemäßen Bedienvorrichtung angesteuert werden.

Nach einer Variante der erfindungsgemäßen Bedienvorrichtung weist diese eine mit der Steuerungsvorrichtung gekoppelte Umschaltvorrichtung auf, wobei die Steuerungsvorrichtung aufgrund eines Betätigen der Umschaltvorrichtung entweder das erste Signal aufgrund des manuellen Bewegens der ersten Eingabevorrichtung erzeugt oder die am Bildschirm dargestellten Informationsinhalte aufgrund eines Betätigens der ersten Eingabevorrichtung ändert. Mittels der Umschaltvorrichtung wird es dem Arzt ermöglicht, in relativ einfacher Weise die erfindungsgemäße Bedienvorrichtung von einem ersten Betriebsmodus, in dem die erste Eingabevorrichtung zum manuellen Bewegen des ersten Roboterarms, in einen zweiten Betriebsmodus zu schalten, in dem die erste Eingabevorrichtung für das Ändern der am Bildschirm dargestellten Informationsinhalte verwendbar ist. Die Umschaltvorrichtung ist z.B. als Fußschalter ausgeführt, was dem Arzt eine relativ einfache Bedienung der erfindungsgemäßen Bedienvorrichtung erlaubt. Die Umschaltvorrichtung kann auch sprachgesteuert ausgeführt sein.

Die Steuerungsvorrichtung kann derart ausgeführt sein, dass sie aufgrund eines Betätigens der Umschaltvorrichtung ein drittes Signal erzeugt, so dass ein zum Behandeln des Lebewesens vorgesehner Roboterarm des medizinischen Arbeitsplatzes eine des manuellen Bewegens entsprechende Bewegung durchführt. Gemäß dieser Variante wird es dem Arzt ermöglicht, die erste Eingabevorrichtung zum Bewegen des ersten oder des zweiten Roboterarms als auch zum Ändern der am Bildschirm dargestellten Informationsinhalte durch relativ einfaches Umschalten zu verwenden.

Nach einer Variante der erfindungsgemäßen Bedienvorrichtung ist deren erste Eingabevorrichtung kraftgeregelt oder kraftrückgekoppelt ausgeführt. In Verbindung mit der Variante, gemäß derer die erste Eingabevorrichtung zum Ändern der am Bildschirm dargestellten Informationsinhalte verwendet wird, ergibt sich ein verbessertes und/oder bequemeres Ändern der dargestellten Informationsinhalte mittels der ersten Eingabevorrichtung, da relevante Information haptisch dargestellt werden kann. Die kraftgeregelte oder kraftrückgekoppelte Ausführungsform kann z.B. ein Navigieren oder ein Darstellen hinterlegter haptischer Information erleichtern.

Ein Ausführungsbeispiel der Erfindung ist exemplarisch in den beigefügten schematischen Zeichnungen dargestellt. Es zeigen:
- Fig. 1: einen medizinischen Arbeitsplatz mit mehreren Roboterarmen und einer Bedienvorrichtung zum manuellen Bewegen der Roboterarme und
- Fig. 2 und 3: mit einem Bildschirm der Bedienvorrichtung dargestellte Informationsinhalte.

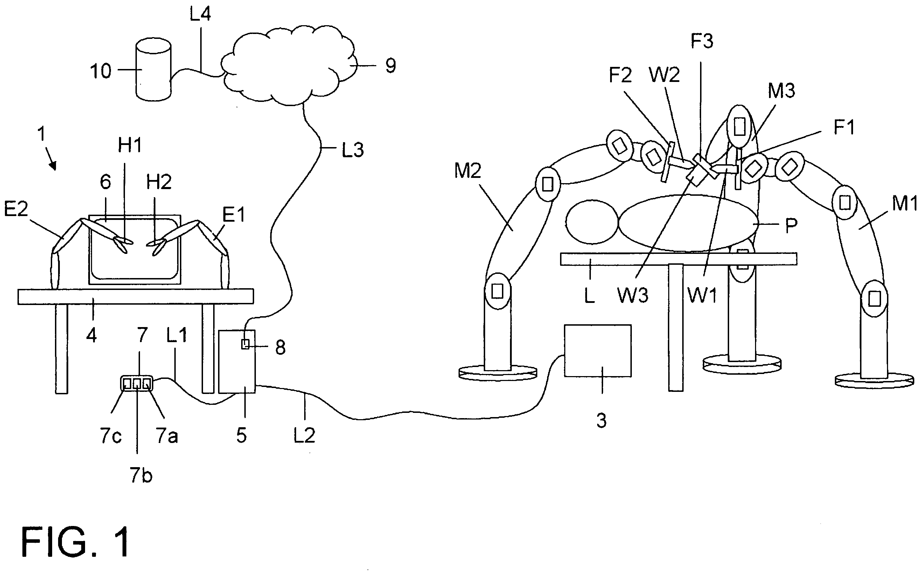

Die Fig. 1 zeigt einen medizinischen Arbeitsplatz, der eine Patientenliege L, mehrere Roboterarme M1-M3 und eine Bedienvorrichtung 1 zum manuellen Bewegen der Roboterarme M1-M3 aufweist. Jeder der Roboterarme M1-M3 weist mehrere mittels Antrieben bewegbare Achsen und eine Befestigungsvorrichtung F1-F3 auf und kann beispielsweise bezüglich sechs Freiheitsgraden bewegt werden.

Im Falle des vorliegenden Ausführungsbeispiels liegt auf der Patientenliege L eine Person P, die mittels der Roboterarme M1-M3 bzw. mittels an den Befestigungsvorrichtungen F1-F3 der Roboterarme M1-M3 befestigten Instrumenten behandelt werden kann. An den Befestigungsvorrichtungen F1, F2 der Roboterarme M1, M2 sind z.B. jeweils medizinische Instrumente W1, W2 und an der Befestigungsvorrichtung F3 des Roboterarms M3 ist beispielsweise eine Kamera W3 befestigt.

Im Falle des vorliegenden Ausführungsbeispiels sind die Antriebe der Roboterarme M-M3, die medizinischen Instrumente W1, W2 und die Kamera W3 in nicht dargestellter Weise mit einem ersten Steuerrechner 3 verbunden. Auf dem ersten Steuerrechner 3 läuft ein Rechnerprogramm, mittels dem der Steuerrechner 3 die Antriebe der Roboterarme M1-M3 derart ansteuern kann, so dass sich die Achsen der Roboterarme M1-M3 in gewünschter Weise derart bewegen, damit die Befestigungsvorrichtungen F1-F3 bzw. die Tool Center Points der medizinischen Instrumente W1, W2 und der Kamera W3 eine gewünschte Lage (Position und Orientierung) einnehmen.

Der medizinische Arbeitsplatz umfasst ferner die Bedienvorrichtung 1. Diese weist im Falle des vorliegenden Ausführungsbeispiels einen zweiten Steuerrechner 5, zwei an einem Tisch 4 angeordnete und in nicht dargestellter Weise mit dem zweiten Steuerrechner 5 verbundene Handeingabevorrichtungen E1, E2, einen in nicht dargestellter Weise mit dem zweiten Steuerrechner 5 verbundenen Bildschirm 6 und eine mittels einer Leitung L1 mit dem zweiten Steuerrechner 5 verbundene Fußumschalteinrichtung 7 auf. Die beiden Steuerrechner 3, 5 können ferner über eine Datenleitung L2 miteinander kommunizieren.

Im Falle des vorliegenden Ausführungsbeispiels weisen die beiden Handeingabevorrichtungen E1, E2 jeweils einen Handgriff H1, H2 und mehrere Achsen auf. Ein in den Figuren nicht näher dargestellter Arzt kann die Handeingabevorrichtungen E1, E2 mittels der Handgriffe H1, H2 manuell bezüglich wenigstens sechs Freiheitsgraden bewegen. Die Handeingabevorrichtungen E1, E2 weisen z.B. den jeweiligen Achsen der Handeingabevorrichtungen E1, E2 zugeordnete und in den Figuren nicht näher dargestellte Winkelsensoren auf, deren Signale dem zweiten Steuerrechner 5 übermittelt werden. Auf dem zweiten Steuerrechner 5 läuft wiederum ein Rechnerprogramm, das aufgrund der von den Handeingabevorrichtungen E1, E2 stammenden Signalen Bewegungen der Handeingabevorrichtungen E1, E2 erkennt. Die beiden Handeingabevorrichtungen E1, E2 können auch mehr als sechs Freiheitsgrade aufweisen.

Im Falle des vorliegenden Ausführungsbeispiels ist die Handeingabevorrichtung E1 dafür vorgesehen, den Roboterarm M1 zu bewegen. Bei einem manuellen Bewegen der Handeingabevorrichtung E1 mittels ihres Handgriffs H1 detektieren die Winkelsensoren der Handeingabevorrichtung E1 Winkeländerungen der relevanten Achsen. Aus den von den Winkelsensoren erzeugten Signalen ermittelt der zweite Steuerrechner 5 entsprechende Bewegungen der Handeingabevorrichtung E1 und übermittelt über die Leitung L2 eine entsprechende Information an den ersten Steuerrechner 3, der daraufhin die Antriebe des Roboterarms M1 derart ansteuert, so dass dessen Befestigungsvorrichtung F1 bzw. dessen Tool Center Point eine der manuellen Bewegung der Handeingabevorrichtung E1 entsprechenden Bewegung durchführt. Die Signale könne auch vor verarbeitet, z.B. gefiltert werden.

Im Falle des vorliegenden Ausführungsbeispiels ist die Handeingabevorrichtung E2 dafür vorgesehen, die beiden anderen Roboterarme M2, M3 zu bewegen. Um einen der beiden Roboterarme M2, M3 für das Bewegen auszuwählen, kann der Arzt die Fußumschalteinrichtung 7 betätigen. Diese weist drei Fußschalter 7a, 7b, 7c auf. Aktiviert der Arzt z.B. mit seinem Fuß den Fußschalter 7a, so kann, ähnlich wie er mit der Handeingabevorrichtung E1 den Roboterarm M1 bewegen kann, mit der Handeingabevorrichtung E2 den Roboterarm M2 bewegen. Aktiviert der Arzt dagegen den Fußschalter 7b, so kann, ähnlich wie er mit der Handeingabevorrichtung E1 den Roboterarm M1 bewegen kann, mit der Handeingabevorrichtung E2 den Roboterarm M3 bewegen.

Im Falle des vorliegenden Ausführungsbeispiels ist am Roboterarm M3 die Kamera W3 befestigt, mit der Bilder vom Operationssitus aufgenommen werden können, so dass der Arzt z.B. eine optische Rückmeldung über die Lagen der Roboterarme M1, M2 und/ oder der den Robotern zugeordneten Instrumente W1 und/ oder W2 erhält. Die den mit der Kamera W3 aufgenommenen Bildern zugeordneten Bilddatensätze werden über die Leitung L2 vom ersten Steuerrechner 3 zum zweiten Steuerrechner 5 übermittelt, damit der zweite Steuerrechner 5 diese Bilder ggf. nach Verarbeitung am Bildschirm 6 darstellten kann.

Im Falle des vorliegenden Ausführungsbeispiels weist der zweite Steuerrechner 5 der Bedienvorrichtung 1 eine Schnittstelle 8 auf, über die der zweite Steuerrechner 5 mit einem Krankenhausdatennetz 9 kommunizieren kann. Dazu ist die Schnittstelle 8 mit dem Krankenhausdatennetz 9 über eine Datenleitung L3 verbunden. Das Krankenhausdatennetz ist wiederum mit einer Datenbank 10 über eine Datenleitung L4 verbunden. In der Datenbank 10 sind u.A. dem Lebewesen P zugeordnete Daten, wie z.B. dessen Patientenakte 11 in elektronischer Form gespeichert.

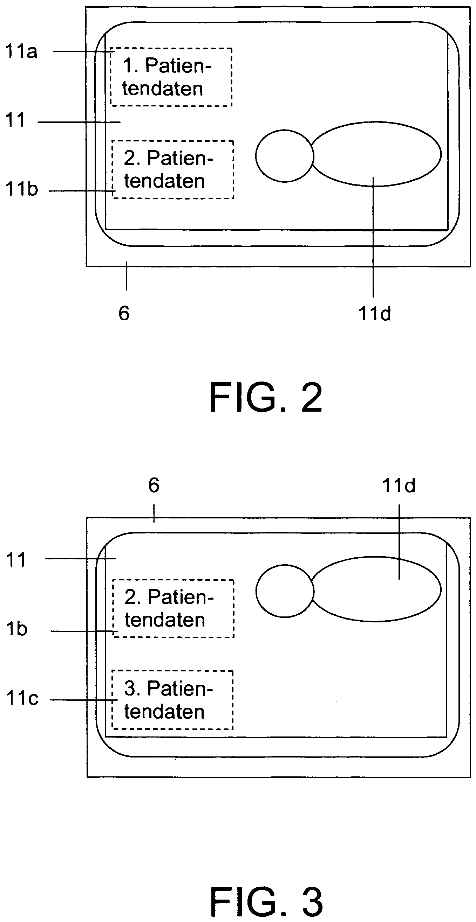

Im Falle des vorliegenden Ausführungsbeispiels ist die Bedienvorrichtung 1 ferner derart eingerichtet, so dass über die Schnittstelle 8 Daten über das Krankenhausdatennetz 9 und insbesondere von der Datenbank 10 abgerufen werden können, so dass z.B. die Patientenakte 11 des Lebewesens P auf dem Bildschirm 6 der Bedienvorrichtung 1 dargestellt werden kann, wie dies in den Figuren 2 und 3 gezeigt ist.

Des Weiteren ist die Bedienvorrichtung 1 derart eingerichtet, so dass durch Betätigen der Fußumschalteinrichtung 7 und insbesondere durch Aktivieren des Fußschalters 7c der Arzt mit der Handeingabevorrichtung E2 anstelle die Roboterarme M2, M3 zu bewegen, Daten von der Datenbank 10 abrufen kann. Dazu sind im Falle des vorliegenden Ausführungsbeispiels die Handeingabevorrichtung E2 bzw. der zweite Steuerrechner 5 derart eingerichtet, so dass der zweite Steuerrechner 5 eine vorgegebene Bewegung der Handeingabevorrichtung E2 als Anweisung zum Abrufen von Daten von der Datenbank 10 erkennt. Ferner ist der zweite Steuerrechner 5 derart eingerichtet, dass, sobald den abgerufenen Daten zugeordnete Informationsinhalte am Bildschirm 6 angezeigt sind, wie z.B. die in den Figuren 2 und 3 gezeigte Patientenakte 11, z.B. ein Ändern der dargestellten Informationsinhalte, z.B. in Form von Navigieren innerhalb der Patientenakte 11 aufgrund einer manuellen Bewegung der Handeingabevorrichtung E2 möglich ist.

Im Falle des vorliegenden Ausführungsbeispiels umfasst die Patientenakte 11 1. Patientendaten 11a, 2. Patientendaten 11b, 3. Patientendaten 11c und ein mittels eines bildgebenden medizinischen Gerätes aufgenommenes Bild 11d des Lebewesens P, das beispielsweise ein Röntgenbild, ein CT- oder ein MR-Bild oder ein Ultraschallbild ist.

Durch ein Bewegen der Handeingabevorrichtung E2 wird es im Falle des vorliegenden Ausführungsbeispiels dem Arzt ermöglicht, die am Bildschirm 6 dargestellte und in den Figuren 2 und 3 gezeigte Patientenakte 11 herunter- und raufzuscrollen, um insbesondere den Text der Patientenakte 11 lesen zu können. Dazu interpretiert der zweite Steuerrechner 5 die aufgrund der manuellen Bewegung der Handeingabevorrichtung E2 erzeugten Signale der Winkelsensoren und steuert den Bildschirm 6 entsprechend an, um das oben genannte Scrollen der in dne Figuren 2 und 3 dargestellten Patientenakte 11 zu ermöglichen. Zusätzlich oder alternativ ist es auch möglich, aufgrund eines manuellen Bewegens der Handeingabevorrichtung E2 die Darstellung des Bildes 11d zu ändern. Dazu ist der zweite Steuerrechner 5 beispielsweise derart eingerichtet, aufgrund eines Bewegens der Handeingabevorrichtung E2 das dargestellte Bild 11d zu drehen, um es in verschiednen Ansichten darzustellen, oder verschiedene Schnitte zu zeigen. Insbesondere ist ein Überlagern mit den von der Kamera W3 aufgenommenen Bildern möglich.

Im Falle des vorliegenden Ausführungsbeispiels sind die beiden Handeingabevorrichtungnen E1, E2 zusätzlich kraftgeregelt und umfassen dazu z.B. in den Figuren nicht dargestellte mit den Achsen der Handeingabevorrichtungen E1, E2 gekoppelte elektrische Antriebe, um z.B. eine Gewichtskraft der Handeingabevorrichtungen E1, E2 auszugleichen oder um dem Arzt eine haptisch Rückmeldung beim Bewegen der Roboterarme M1-M3 und/ oder Navigieren und/oder Verändern der Patientenakte 11 zu ermöglichen.

Zusätzlich oder alternativ ist es auch möglich, auch die Handeingabevorrichtung E1 umzuschalten, so dass mit dieser Daten von der Datenbank 10 über die Schnittstelle 8 des zweiten Steuerrechners 5 abgerufen werden können und/oder um die Eingabevorrichtung E1 zum Ändern der am Bildschirm 6 dargestellten Informationsinhalte, wie die Patientenakte 11, zu verwenden.

## Patentansprüche

1. Bedienvorrichtung zum manuellen Bewegen eines Roboterarms eines medizinischen Arbeitsplatzes, aufweisend
- eine Steuerungsvorrichtung (5), die eingerichtet ist, ein zum Steuern einer Bewegung eines zum Behandeln eines Lebewesens (P) vorgesehenen ersten Roboterarms (M2) vorgesehenes erstes Signal zu erzeugen, und die eine Schnittstelle (8) aufweist, die vorgesehen ist, mit einem Krankenhausdatennetz (9) verbunden zu werden,
- eine mit der Steuerungsvorrichtung (5) gekoppelte manuelle erste Eingabevorrichtung (E2), wobei die Steuerungsvorrichtung (5) das erste Signal aufgrund eines manuellen Bewegens der ersten Eingabevorrichtung (E2) erzeugt, so dass der erste Roboterarm (M2) eine des manuellen Bewegens entsprechende Bewegung durchführt, und
- einen Bildschirm (6),
**dadurch gekennzeichnet, dass** die Steuerungsvorrichtung (5) eingerichtet ist, dem Lebewesen (P) zugeordnete, vor einer Behandlung des Lebewesens (P) mit dem ersten Roboterarm (M2) erstellte Daten über die Schnittstelle (8) und das Krankenhausdatennetz (9) abzurufen und den Daten zugeordnete Informationsinhalte (11) am Bildschirm (6) darzustellen, und die Steuerungsvorrichtung (5) und deren erste Eingabevorrichtung (E2) eingerichtet sind, die am Bildschirm (6) dargestellten Informationsinhalte (11) mittels der ersten Eingabevorrichtung (E2) zu ändern.

2. Bedienvorrichtung nach Anspruch 1, bei dem die Informationsinhalte Bilder (11d) umfassen, welche insbesondere mit einem bildgebenden medizinischen Gerät aufgenommen wurden.

3. Bedienvorrichtung nach Anspruch 1 oder 2, bei der das Ändern der am Bildschirm (6) dargestellten Informationsinhalte ein Überlagern mit von einer Kamera (W3) aufgenommener Bilder umfasst, wobei die Kamera (W3) am ersten Roboterarm oder an einem zweiten Roboterarm befestigt ist, oder wobei der erste oder der zweite Roboterarm mit einem medizinischen Instrument versehen ist, das die Kamera umfasst.

4. Bedienvorrichtung nach einem der Ansprüche 1 bis 3, bei der eine Änderung der dargestellten Informationsinhalte eine Änderung deren Darstellung, insbesondere ein Navigieren innerhalb der Informationsinhalte, und/oder eine Änderung der den Informationsinhalten zugeordneten Daten umfasst.

5. Bedieneinrichtung nach einem der Ansprüche 1 bis 4, deren erste Eingabevorrichtung (E2) mehr Freiheitsgrade als der erste Roboterarm (M2) aufweist.

6. Bedienvorrichtung nach einem der Ansprüche 1 bis 5, aufweisend eine mit der Steuerungsvorrichtung (5) gekoppelte manuelle zweite Eingabevorrichtung (E1), wobei die Steuerungsvorrichtung (5) eingerichtet ist, aufgrund eines manuellen Bewegens der zweiten Eingabevorrichtung (E1) ein zweites Signal zu erzeugen, aufgrund dessen ein zur Behandlung des Lebewesens (P) vorgesehener zweiter Roboterarm (M1) eine des manuellen Bewegens der zweiten Eingabevorrichtung (E1) entsprechenden Bewegung durchführt, wobei die Steuerungsvorrichtung (5) und die zweite Eingabevorrichtung (E1) eingerichtet sind, die am Bildschirm (6) dargestellten Informationsinhalte (11) mittels der zweiten Eingabevorrichtung (E1) zu ändern.

7. Bedienvorrichtung nach einem der Ansprüche 1 bis 6, aufweisend eine mit der Steuerungsvorrichtung (5) gekoppelte Umschaltvorrichtung (7), wobei die Steuerungsvorrichtung (5) aufgrund eines Betätigen der Umschaltvorrichtung (7) entweder das erste Signal aufgrund des manuellen Bewegens der ersten Eingabevorrichtung (E2) erzeugt oder die am Bildschirm (6) dargestellten Informationsinhalte (11) aufgrund eines Betätigens der ersten Eingabevorrichtung über die Schnittstelle (8) abruft und/oder ändert.

8. Bedienvorrichtung nach Anspruch 7, deren Steuerungsvorrichtung (5) derart ausgeführt ist, dass sie aufgrund eines Betätigens der Umschaltvorrichtung (7) ein drittes Signal erzeugt, so dass ein zum Behandeln des Lebewesens (8) vorgesehener dritter Roboterarm (M3) eine des manuellen Bewegens entsprechenden Bewegung durchführt.

9. Bedienvorrichtung nach einem der Ansprüche 1 bis 8, deren erste Eingabevorrichtung (E2) kraftgeregelt oder kraftrückgekoppelt ausgeführt ist.

10. Bedienvorrichtung nach einem der Ansprüche 1 bis 9, wobei die dem Lebewesen (P) zugeordneten Daten in einer Datenbank (10) des Krankenhausdatennetzes (9) gespeichert sind und die Steuerungsvorrichtung (5) eingerichtet ist, diese Daten von der Datenbank (10) über die Schnittstelle (8) abzurufen.

11. Bedienvorrichtung nach einem der Ansprüche 1 bis 10, bei der die Daten einer Krankenakte (11) des Lebewesens (P) zugeordnet sind.

12. Bedienvorrichtung nach einer der Ansprüche 1 bis 11, bei der die Daten vor einer Behandlung des Lebewesens (P) mit dem ersten Roboterarm (M2) erstellt wurden.

13. Medizinischer Arbeitsplatz, aufweisend eine Bedienvorrichtung (1) nach einem der Ansprüche 1 bis 12 und wenigstes einen zum Behandeln des Lebewesens (P) vorgesehenen medizinischen Roboterarm (M1-M3), dessen Bewegung mittels der Bedienvorrichtung (1) manuell steuerbar ist.

## Claims

1. Operating device for manually moving a robot arm of a medical workstation, comprising
- a control device (5) which is set up to generate a first signal intended for controlling a movement of a first robot arm (M2) provided for treating a living being (P), and which has an interface (8) which is provided to be connected to a hospital data network (9),
- a manual first input device (E2) coupled to the control device (5), wherein the control device (5) generates the first signal on the basis of a manual movement of the first input device (E2), so that the first robot arm (M2) executes a movement corresponding to the manual movement, and
- a screen (6),
**characterised in that** the control device (5) is configured to retrieve data via the interface (8) and the hospital data network (9) assigned to the living being (P) and created before a treatment of the living being (P) with the first robot arm (M2) and to represent informational content (11) assigned to the data on the screen (6), and the control device (5) and its first input device (E2) are configured to change the informational content (11) shown on the screen (6) by means of the first input device (E2).

2. Operating device according to claim 1, wherein the informational content comprises images (11d) which have been captured in particular by an imaging medical device.

3. Operating device according to claim 1 or 2, wherein the change of informational content represented on the screen (6) comprises overlayering with images captured by a camera (W3), wherein the camera (W3) is secured to the first robot arm or to a second robot arm, or wherein the first or the second robot arm is provided with a medical instrument which comprises the camera.

4. Operating device according to any of claims 1 to 3, wherein a change to the shown informational content comprises a change to its presentation, in particular navigation within the informational content and/or a change of the data assigned to the informational content.

5. Operating device according to any of claims 1 to 4, the first input device (E2) of which has more degrees of freedom than the first robot arm (M2).

6. Operating device according to any of claims 1 to 5, having a manual second input device (E1) coupled to the control device (5), the control device (5) being set up to generate a second signal on the basis of a manual movement of the second input device (E1), on the basis of which a second robot arm (M1) provided for treating the living being (P) executes a movement corresponding to the manual movement of the second input device (E1), wherein the control device (5) and the second input device (E1) are set up to change the informational content (11) displayed on the screen (6) by means of the second input device (E1).

7. Operating device according to any of claims 1 to 6, having a switching device (7) coupled to the control device (5), wherein the control device (5) on the basis of activating the switching device (7) either generates the first signal on the basis of the manual movement of the first input device (E2) or retrieves and/or changes via the interface (8) the informational content (11) displayed on the screen (6) on the basis of an activation of the first input device.

8. Operating device according to claim 7, the control device (5) of which is designed such that it generates a third signal on the basis of activating the switching device (7), so that a third robot arm (M3) provided for treating the living being (8) executes a movement corresponding to the manual movement.

9. Operating device according to any of claims 1 to 8, the first input device (E2) of which is designed to be controlled by force or feedback coupled by force.

10. Operating device according to any of claims 1 to 9, wherein the data assigned to the living being (P) are stored in a database (10) of the hospital data network (9) and the control device (5) is set up to retrieve said data from the database (10) via the interface (8).

11. Operating device according to any of claims 1 to 10, wherein the data are assigned to a clinical record (11) of the living being (P).

12. Operating device according to any of claims 1 to 11, wherein the data have been created prior to a treatment of the living being (P) with the first robot arm (M2).

13. Medical workstation, having an operating device (1) according to any of claims 1 to 12 and at least one medical robot arm (M1-M3) provided for treating the living being (P), the movement of which robot arm can be controlled manually by means of the operating device (1).

## Revendications

1. Dispositif de manipulation pour déplacer manuellement un bras de robot d'un poste de travail médical, présentant
- un dispositif de commande (5) qui est configuré pour générer un premier signal prévu pour commander un mouvement d'un premier bras de robot (M2) prévu pour traiter un être vivant (P) et qui présente une interface (8) qui est prévue pour être connectée à un réseau informatique hospitalier (9),
- un premier dispositif d'entrée manuelle (E2) couplé au dispositif de commande (5), le dispositif de commande (5) générant le premier signal sur la base d'un mouvement manuel du premier dispositif d'entrée (E2) de telle sorte que le premier bras de robot (M2) exécute un mouvement correspondant au déplacement manuel,
et
- un écran (6),
**caractérisé en ce que** le dispositif de commande (5) est configuré pour extraire des données associées à la créature vivante (P) et générées avant le traitement de la créature vivante (P) avec le premier bras du robot (M2) via l'interface (8) et le réseau de données hospitalier (9) et pour afficher sur l'écran (6) des contenus d'informations (11) associés aux données, et le dispositif de commande (5) et son premier dispositif d'entrée (E2) sont configurés pour modifier les contenus d'informations (11) affichés sur l'écran (6) au moyen du premier dispositif d'entrée (E2).

2. Dispositif de manipulation selon la revendication 1, dans lequel les contenus d'informations comprennent des images (11d) qui ont été prises en particulier avec un dispositif médical d'imagerie.

3. Dispositif de manipulation selon la revendication 1 ou 2, dans lequel la modification des contenus d'informations affichés sur l'écran (6) comprend une superposition d'images prises par une caméra (W 3), la caméra (W 3) étant fixée au premier bras du robot ou à un second bras du robot, ou bien le premier ou le second bras du robot étant équipé d'un instrument médical comprenant la caméra.

4. Dispositif de manipulation selon l'une des revendications 1 à 3, dans lequel une modification des contenus d'informations affichés comprend une modification de leur affichage, en particulier une navigation au sein des contenus d'informations, et/ou une modification des données associées aux contenus d'informations.

5. Dispositif de manipulation selon l'une des revendications 1 à 4, dont le premier dispositif d'entrée (E2) présente plus de degrés de liberté que le premier bras du robot (M2).

6. Dispositif de manipulation selon l'une des revendications 1 à 5, présentant un deuxième dispositif d'entrée manuel (EI) couplé au dispositif de commande (5), le dispositif de commande (5) étant configuré pour générer un deuxième signal sur la base d'un déplacement manuel du deuxième dispositif d'entrée (EI), sur la base duquel un deuxième bras de robot (MI) prévu pour le traitement de l'être vivant (P) exécute un mouvement correspondant au déplacement manuel du deuxième dispositif d'entrée (E1), le dispositif de commande (5) et le deuxième dispositif d'entrée (E1) étant configurés pour modifier les contenus d'informations (11) affichés sur l'écran au moyen du deuxième dispositif d'entrée (E1).

7. Dispositif de manipulation selon l'une des revendications 1 à 6, présentant un dispositif de commutation (7) couplé au dispositif de commande (5), dans lequel le dispositif de commande (5), en raison d'un actionnement du dispositif de commutation (7), soit génère le premier signal sur la base d'un déplacement manuel du premier dispositif d'entrée (E2), soit extraie et/ou modifie les contenus d'informations (11) affichés sur l'écran (6) lors de l'actionnement du premier dispositif d'entrée via l'interface (8) en raison d'un actionnement du premier dispositif d'entrée.

8. Dispositif de manipulation selon la revendication 7, dont le dispositif de commande (5) est configuré de telle sorte qu'il génère un troisième signal en raison d'un actionnement du dispositif de commutation (7), de telle sorte qu'un troisième bras de robot (M3) prévu pour traiter l'être vivant (8) effectue un déplacement correspondant au mouvement manuel.

9. Dispositif de manipulation selon l'une des revendications 1 à 8, dont le premier dispositif d'entrée (E2) est réglé en force ou en retour de force par couplage.

10. Dispositif de manipulation selon l'une des revendications 1 à 9, dans lequel les données associées à l'être vivant (P) sont mémorisées dans un fichier de base de données (10) du réseau de données hospitalier (9), et le dispositif de commande (5) est configuré pour extraire lesdites données depuis la base de données (10) via l'interface (8).

11. Dispositif de manipulation selon l'une des revendications 1 à 10, dans lequel les données sont associées à un dossier médical (11) de l'être vivant (P).

12. Dispositif de manipulation selon l'une des revendications 1 à 11, pour lequel les données ont été générées avant le traitement de l'être vivant (P) avec le premier bras du robot (M2).

13. Poste de travail médical présentant un dispositif de dispositif de manipulation (1) selon l'une des revendications 1 à 12 et au moins un bras de robot médical (MI -M3) prévu pour traiter l'être vivant (P), dont le mouvement peut être commandé manuellement au moyen du dispositif de manipulation (1).
